# EUROPÄISCHE PATENTANMELDUNG

(11) **EP 1 656 886 A1**
(43) Veröffentlichungstag der Anmeldung: **17.05.2006**
(21) Anmeldenummer: 04027137.1
(22) Anmeldetag: 15.11.2004
(51) Int. Cl.: A61B 10/00, A61B 17/32

(54) **Vorrichtung zum Ausschneiden von Gewebeproben**

(71) Anmelder: Roche Diagnostics GmbH, 68305 Mannheim (DE); F.HOFFMANN-LA ROCHE AG, 4070 Basel (CH)
(72) Erfinder: Die Erfindernennung liegt noch nicht vor

(57) **Zusammenfassung**

Die vorliegende Erfindung betrifft eine Vorrichtung zum Abtrennen eines Gewebeteils aus Hirnmaterial umfassend eine kreisrunde Klinge, die sich als Wand eines zylindrischen oder im Wesentlichen zylindrischen Hohlkörpers fortsetzt, an dessen Ende ein senkrecht zur Zylinderachse angeordnetes Gitter angebracht ist. Der Erfindung betrifft weiterhin die Verwendung der Vorrichtung in einer Methode zum Abtrennen eines Gewebeteils aus Hirnmaterial sowie einen Kit enthaltend eine oder mehrere erfindungsgemäße Vorrichtungen.

## Beschreibung

Gegenstand der vorliegenden Erfindung ist eine Schneidevorrichtung, mit deren Hilfe eine quantitativ definierte Gewebeprobe aus einer größeren Gewebeprobe oder einem Gewebeverbund, bevorzugt Hirngewebe, entnommen werden kann. Eine bevorzugte Ausführungsform ist eine Schneidevorrichtung für den einmaligen Gebrauch. Die Erfindung ist weiterhin gerichtet auf eine Methode zur Entnahme von quantitativ definierten Gewebeproben aus einer größeren Gewebeprobe oder einem Gewebeverbund unter Verwendung der Schneidevorrichtung.

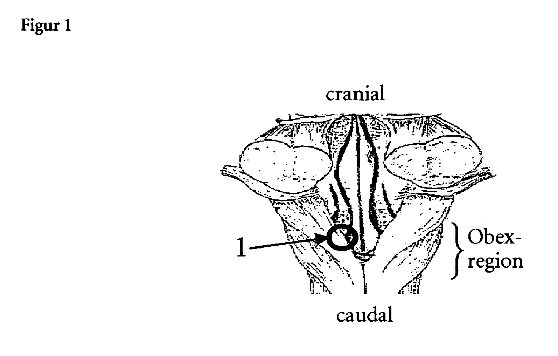

Gegenwärtig werden Rinder, die ein bestimmtes Alter überschritten haben, nach der Schlachtung mit Hilfe eines Tests auf BSE (Prioneninfektion) untersucht. Der Schlachtkörper wird erst zur weiteren Verarbeitung freigegeben, wenn das Testergebnis vorliegt. Ein Teil des Stammhirns (Medulla oblongata), bevorzugt die Obexregion (Figur 1), dient als Ausgangsmaterial zur Entnahme einer Gewebeprobe, mithilfe derer der Test durchgeführt wird. Im Schlachthaus wird ein Teil des Stammhirns entnommen. Durch das Foramen magnum, d.h. durch die große Öffnung am hinteren Schädel, durch die das Rückenmark austritt, wird der Obex zusammen mit angrenzenden Bereichen mittels eines speziell geformten Löffels entnommen und in einen Spezialbehälter überführt. In dem Behälter gelangt das Hirnmaterial in ein Untersuchungslabor, wo die eigentliche Entnahme der Probe zur Feststellung einer Prioneninfektion erfolgt.

Die gegenwärtig auf dem Markt befindlichen Testsysteme machen es erforderlich, dass der Nachweis mit einer definierten Menge Probenmaterial vorgenommen wird. Die Mengenbestimmung erfolgt überlicherweise durch Auswiegen. Hierbei müssen manuelle Arbeitsschritte durchgeführt werden, bei denen potentiell infektiöses Material unter hohen Anforderungen in Bezug auf biologische Sicherheit gehandhabt wird. Insbesondere unter Berücksichtigung der Arbeitssicherheit ist es wünschenswert, die Arbeitsschritte mit unverschlossenem Hirnmaterial zu begrenzen. Konkret wäre es von Vorteil, die Mengenbestimmung schneller und einfacher vornehmen zu können, da einem Testsystem auf diese Weise pro Zeiteinheit mehr Proben zugeführt werden können. Dies hätte eine positive Auswirkung auf den im Labor erzielbaren Probendurchsatz.

Eine Aufgabe der vorliegenden Erfindung war es daher, eine Vorrichtung bereit zu stellen, mit deren Hilfe eine quantitativ definierte Gewebeprobe aus potentiell infektiösem Hirnmaterial schnell und zuverlässig und unter reduziertem manuellen Aufwand gewonnen werden kann. Eine weitere Aufgabe bestand darin, eine Methode bereit zu stellen, bei der die Anzahl der manuellen Schritte, die zu einer quantitativ definierten Gewebeprobe führen, verringert ist. Eine weitere Aufgabe der Erfindung war es, eine Methode bereitzustellen, bei der die Schritte, die zu einer quantitativ definierten Gewebeprobe führen, vereinfacht und beschleunigt werden.

Erfindungsgemäß wurde das Problem gelöst durch die Bereitstellung einer Vorrichtung zum Ausschneiden eines Gewebeteils aus Hirnmaterial (im Folgenden auch Schneidevorrichtung genannt). Eine erste bevorzugte Ausführungsform der Erfindung ist eine Vorrichtung zum Abtrennen eines Gewebeteils aus Hirnmaterial umfassend eine kreisrunde Klinge, die sich als Wand eines zylindrischen oder im Wesentlichen zylindrischen Hohlkörpers fortsetzt, an dessen Ende ein senkrecht zur Zylinderachse angeordnetes Gitter angebracht ist. Eine weitere bevorzugte Ausführungsform ist eine Vorrichtung, dadurch gekennzeichnet, dass die Vorrichtung zusätzlich einen Griff umfasst, der jenseits des Gitters am Zylinder der Klinge ansetzt und diesen in Richtung der Längsachse verlängert. Eine weitere bevorzugte Ausführungsform ist eine Vorrichtung, dadurch gekennzeichnet, dass der Griff ein offener Hohlkörper ist. Eine weitere bevorzugte Ausführungsform ist eine Vorrichtung, dadurch gekennzeichnet, dass der Griff eine Verbreiterung mit der Oberfläche eines Kegelstumpfes umfasst, dessen Enden kreisförmig sind, wobei das Ende mit dem geringeren Kreisdurchmesser jenseits des Gitters bündig am Zylinder der Klinge ansetzt und das Ende mit dem größeren Durchmesser bündig an einem zylindrischen oder im Wesentlichen zylindrischen Hohlkörper ansetzt. Eine weitere bevorzugte Ausführungsform ist eine Vorrichtung, dadurch gekennzeichnet, dass der Griff folgendes umfasst: (a) einen ersten zylindrischen Hohlkörper, der jenseits des Gitters bündig am Zylinder der Klinge ansetzt und diesen in Richtung der Längsachse verlängert, (b) eine Verbreiterung mit der Oberfläche eines Kegelstumpfes, dessen Enden kreisförmig sind, wobei das Ende mit dem geringeren Kreisdurchmesser jenseits des Gitters am Zylinder von (a) ansetzt und das Ende mit dem größeren Durchmesser bündig an (c) einem zylindrischen oder im Wesentlichen zylindrischen Hohlkörper ansetzt. Eine weitere bevorzugte Ausführungsform ist eine Vorrichtung, dadurch gekennzeichnet, dass der Abstand zwischen Klingenkante und Gitter zwischen 5 mm und 20 mm beträgt.

Eine weitere bevorzugte Ausführungsform ist die Verwendung einer Vorrichtung zum Abtrennen eines Gewebeteils aus Hirnmaterial. Eine weitere bevorzugte Ausführungsform ist eine Methode zum Abtrennen eines Gewebeteils aus Hirnmaterial, umfassend die Schritte: (a) Bereitstellen eines Stückes Stammhirn enthaltend die Obex-Region, wobei das Stück Stammhirn mit der ventralen Seite auf eine glatte, feste Unterlage gelegt wird; gefolgt von (b) senkrechtes Aufsetzen der Klinge einer Vorrichtung gemäß einem der Ansprüche 1 bis 6 auf einen vorbestimmten Ort der Obex-Region; gefolgt von (c) Drücken der Klinge in senkrechter Richtung in das Hirnmaterial unter Durchführung von Drehbewegungen, bis die Klinge komplett Kontakt mit der Unterlage hat gefolgt von (d) Herausziehen der Vorrichtung aus dem Stück Stammhirn und Entnehmen des darin enthaltenen Gewebeteils. Eine weitere bevorzugte Ausführungsform ist eine Methode umfassend folgenden zusätzlichen Schritt nach Schritt (c): (c') Drücken der Vorrichtung gegen die Unterlage und Anheben des Stückes Stammhirn, wobei zähe oder faserige Gewebebestandteile von dem von der Vorrichtung umschlossenen Gewebekern abgetrennt werden. Eine weitere bevorzugte Ausführungsform ist ein Kit enthaltend eine oder mehrere erfindungsgemäße Vorrichtungen, eine Gebrauchsvorschrift und Verpackungsmaterial enthaltend die vorgenannten Gegenstände.

### Eingehende Beschreibung

Die Schneidevorrichtung umfasst eine kreisrunde Klinge, die sich als Wand eines zylindrischen oder im Wesentlichen zylindrischen Hohlkörpers fortsetzt, an dessen Ende ein senkrecht zur Zylinderachse angeordnetes Gitter angebracht ist. Weiterhin umfasst die Schneidevorrichtung einen Griff, der die Zylinderachse jenseits des Gitters in Richtung der Längsachse (verlaufend von der Schneidkante zum Gitter) verlängert. In einer besonders bevorzugten Ausführungsform ist der Griff ein Hohlkörper, der besonders bevorzugt nach beiden Seiten hin offen ist.

In einer bevorzugten Ausführungsform enthält das Gitter mindestens 5 Elemente mit Öffnungen. Bevorzugt sind 15 bis 30 Elemente mit Öffnungen.

In einer ersten bevorzugten Ausführungsform besteht der Griff aus einem zylindrischen oder im Wesentlichen zylindrischen Hohlkörper, der jenseits des Gitters am Zylinder der Klinge ansetzt.

In einer weiteren bevorzugten Ausführungsform umfasst der Griff eine Verbreiterung mit der Oberfläche eines Kegelstumpfes, dessen Enden kreisförmig sind, wobei das Ende mit dem geringeren Kreisdurchmesser jenseits des Gitters bündig am Zylinder der Klinge ansetzt und das Ende mit dem größeren Durchmesser bündig an einem zylindrischen oder im Wesentlichen zylindrischen Hohlkörper ansetzt.

In einer weiteren bevorzugten Ausführungsform umfasst der Griff (a) einen ersten zylindrischen Hohlkörper, der jenseits des Gitters bündig am Zylinder der Klinge ansetzt und diesen in Richtung der Längsachse verlängert, (b) eine Verbreiterung mit der Oberfläche eines Kegelstumpfes, dessen Enden kreisförmig sind, wobei das Ende mit dem geringeren Kreisdurchmesser jenseits des Gitters am Zylinder von (a) ansetzt und das Ende mit dem größeren Durchmesser bündig an (c) einem zylindrischen oder im Wesentlichen zylindrischen Hohlkörper ansetzt.

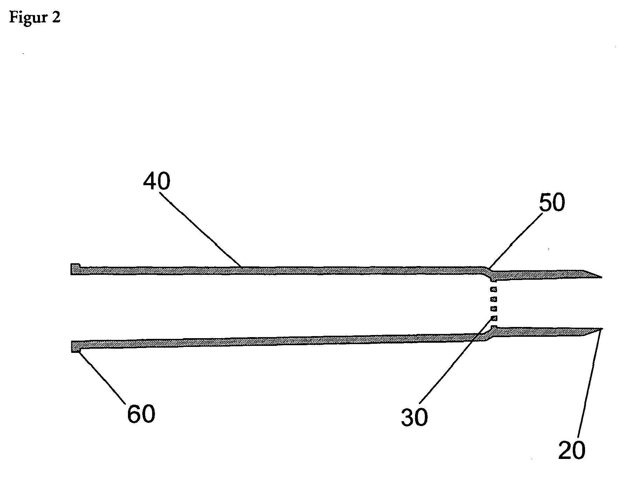

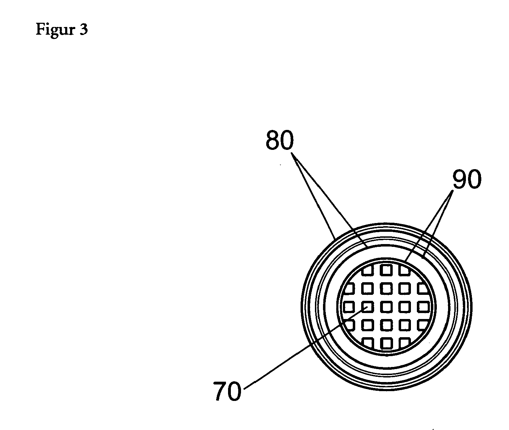

Figur 2 und Figur 3 zeigen Einzelheiten einer bevorzugten Ausführungsform der erfindungsgemäßen Schneidevorrichtung.

Da die Schneidevorrichtung für die Bearbeitung potentiell infektiösen Hirnmaterials vorgesehen ist, dient sie dem Anwender bevorzugt für einen einmaligen Gebrauch (Einmalgegenstand). Einmalgegenstände sind in großer Stückzahl vorteilhaft durch Spritzgussverfahren herzustellen. Verschiedene Grundstoffe, die in Spritzgussverfahren eingesetzt werden, sind dem Fachmann bekannt. Aufgrund der benötigten Klingenfunktion der Schneidkante sind härtere Kunststoffe mehr bevorzugt als weichere. Bevorzugt hierzu ist ein Kunststoffmaterial aus der Gruppe der Polycarbonate, besonders bevorzugt ist der Kunststoff Makrolon® . Am meisten bevorzugt ist Makrolon® 2458.

Mit der Schneidevorrichtung kann wie weiter unten beschrieben aus einem größeren Gewebeverband ein Gewebeteil als zylindrischer Kern herausgeschnitten werden. Der bevorzugte Abstand zwischen Klingenkante und Gitter entspricht im Wesentlichen der Stärke des zu durchschneidenden Materials. Der bevorzugte Durchmesser des durch die Klinge gebildeten Kreises und damit des Durchmessers des Innenraums richtet sich nach der benötigten Probenmenge.

Eine besonders bevorzugte Ausführungsform des Schneidewerkzeugs dient zum Herausschneiden eines zylindrischen oder im Wesentlichen zylindrischen Gewebekerns aus dem als Obex bezeichneten Bereich des Stammhirns, wobei der Gewebekern aus dem Obex in dorso-ventraler Richtung an der in Figur 1 mit 1 bezeichneten Stelle (Entnahmestelle) geschnitten wird, wie weiter unten beschrieben.

In der Regel kommen die Stammhirnstücke, aus denen Gewebekerne als Proben entnommen werden sollen, von etwa gleich alten Tieren. Das bedeutet, dass sich die Ausmaße der Obexregion dieser Tiere nur wenig unterscheiden. Infolgedessen reicht es üblicherweise, für einen gegebenen Zweck, beispielsweise die Probennahme aus Stammhirnstücken von Rindern mit im Wesentlichen gleichem Alter, die Schneidevorrichtung in einer einzigen bestimmten Größe (bezogen auf den damit herstellbaren Gewebekern) zur Verfügung zu stellen.

Der Abstand zwischen Klingenkante und Gitter beträgt für die Entnahme von Gewebekernen daher bevorzugt zwischen 5 mm und 20 mm, besonders bevorzugt zwischen 10 mm und 15 mm, ganz besonders bevorzugt etwa 12 mm. Der Durchmesser des durch die Klinge gebildeten Kreises beträgt bevorzugt zwischen 3 mm und 10 mm, besonders bevorzugt zwischen 5 und 7 mm, ganz besonders bevorzugt 5,8 mm.

Die bevorzugte Wanddicke der Klingenwand und der Gitterelemente beträgt bevorzugt zwischen 0.3 mm und 1,5 mm, besonders bevorzugt zwischen 0,5 mm und 1 mm, noch mehr bevorzugt zwischen 0,6 mm und 0,8 mm. Die Kante der kreisrunden Klinge ist scharfkantig, wobei sich die Schneidkante von außen nach innen hin in einem Winkel von zwischen 10° und 30° (1° wird verstanden als 1/360 des Kreisumfangs) verjüngt. Ein bevorzugter Verjüngungswinkel beträgt zwischen 15° und 25°, besonders bevorzugt ist ein Verjüngungswinkel von etwa 20°.

Die Wanddicke des Griffs ist nur insofern von Belang, als dass sie eine bruchfreie Handhabung der Schneidevorrichtung gewährleisten muss. Eine bevorzugte Wanddicke im Griffbereich ist zwischen 0,5 mm und 2 mm.

In manchen bevorzugten Fällen ist der Innenraum des schneidenden Teils der Vorrichtung im Wesentlichen zylindrisch. Hierunter wird verstanden, dass sich der Innenraum von der Schneidkante zum Gitter hin verjüngt. Diese Anordnung ist vorteilhaft, wenn die Schneidevorrichtung aus einem Kunststoffmaterial mittels eines Spritzgussverfahrens hergestellt wird. Typischerweise beträgt der Winkel der Verjüngung zwischen 0,5° und 10°, bevorzugt zwischen 0,5° und 5°, besonders bevorzugt 1°. Das Volumen des Innenraums von der Klingenkante bis zum Gitter entspricht dem Volumen des Gewebekerns, der mit der Schneidevorrichtung ausgeschnitten wird. Das Volumen des Innenraums richtet sich bevorzugt nach der Menge von Hirnmaterial, das für Untersuchungszwecke benötigt wird. Das Volumen des Innenraums beträgt in einer bevorzugten Ausführungsform der Schneidevorrichtung zwischen 100 µl und 200 µl; ein Volumen zwischen 125 µl und 175 µl ist mehr bevorzugt. Besonders bevorzugt ist ein Volumen von ungefähr 150 µl.

Zur Entnahme eines zylindrischen oder im Wesentlichen zylindrischen Gewebekerns mit definierter Menge, also einer quantitativ definierten Probe, als wird das Stück Stammhirn mit dessen ventraler Seite auf eine waagerechte feste Unterlage gelegt, so dass die dorsale Seite nach oben weist, entsprechend der in Figur 1 gezeigten Aufsicht auf die dorsale Seite der Medulla oblongata. Nähere Informationen zur Entnahmestelle finden sich unter der Internetadresse http://niah.naro.affrc.go.jp/disease/bse/retest_prionics.html. An der mit (1) bezeichneten Stelle wird die kreisförmige Klinge der Schneidevorrichtung unter Durchführung von Drehbewegungen senkrecht in das Hirnmaterial hineingedrückt, bis die Klinge komplett Kontakt mit der Unterlage hat. Hierdurch wird eine zylindrische oder im Wesentlichen zylindrische Gewebemasse (Gewebekern) aus der Obexregion abgetrennt. Dieser Arbeitsgang erfolgt üblicherweise manuell.

Das in der Schneidevorrichtung angebrachte Gitter begrenzt einerseits die Aufnahme von Gewebematerial, andererseits erlaubt sie das Entweichen von Luft aus dem Inneren des durch die kreisförmige Klinge gebildeten Raumes.

Mit Hilfe einer Pinzette wird nun das Stück Stammhirn in Griffrichtung geschoben und hierdurch unter Umständen verbliebene zähe Verbindungen von Gewebeteilen zum Gewebekern getrennt. Die Schneidevorrichtung wird aus dem Stammhirnstück heraus gezogen und der Gewebekern wird mit Hilfe einer Pinzette aus dem Innenraum entnommen und in ein Untersuchungsgefäß überführt.

Das im Stanzwerkzeug angebrachte Gitter hat bei diesem Schritt eine weitere Funktion. Es verhindert bei der Entnahme des Gewebekerns das Entstehen eines Unterdrucks, der das weiche Gewebe zurückhalten und zu dessen Zerreißen führen würde. Auf diese Weise lassen sich auch weiche oder fragile Gewebeproben entnehmen, ohne dass eine erhöhte Kontaminationsgefahr gegeben ist.

Nach der Benutzung wird die Schneidevorrichtung desinfiziert und entsorgt. Zu diesem Zweck wird die Schneidevorrichtung bevorzugt autoklaviert. Die geringe Größe der erfindungsgemäßen Schneidevorrichtung ergibt eine geringe zu entsorgende Abfallmenge. Eine andere oder auch sich anschließende bevorzugte Entsorgungsart ist das Verbrennen der Schneidevorrichtung.

Die folgenden Beispiele, Publikationen und die Abbildungen erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.

### Beschreibung der Figuren

- Figur 1: Dorsale Ansicht auf den Abschnitt eines Rinder-Stammhirns, der die Obex-Region enthält. Cranial: schädelseitig, caudal: rückenmarksseitig.
- Figur 2: Die Figur zeigt den längs auf der mittleren Längsachse verlaufenden Querschnitt einer erfindungsgemäßen Vorrichtung. (20) Schneidkante; (30) Gitter; (40) Griff; (50) Verbreiterungselement; (60) am Griff angebrachter Wulst. Der Griff ist hohl und sowohl zum Gitter hin als auch auf der dem Gitter gegenüber liegenden Seite offen.
- Figur 3: Aufsicht entlang der mittleren Längsachse auf das Gitter; der Blick erfolgt durch den Griff der in Figur 1 dargestellten Vorrichtung hindurch, wobei der Punkt des Betrachters auf der mittleren Längsachse liegt. (70) Gitterelement mit Öffnung; (80) Wulst und Griff, entsprechend (40) und (60) in Figur 1; (90) Verbreiterungselement, entsprechend (50) in Figur 1.

### Beispiel 1

### Entnahme einer Gewebeprobe aus Obex eines Schlachtrinds

Im Schlachthaus wird die Medulla oblongata oder ein die Obex-Region enthaltender Teil durch das Foramen magnum mit Hilfe eines speziell geformten Löffels entnommen und in einem Spezialbehälter gelagert. Im Labor wird mit Hilfe der erfindunsgemäßen Vorrichtung ein in etwa zylindrisches Gewebestück ausgeschnitten und mit der Vorrichtung aus dem Stück Stammhirn herausgezogen. Im Anschluss wird das ausgeschnittene Gewebestück mittels einer Pinzette der Vorrichtung entnommen und der Analyse zugeführt, um gegebenenfalls das Vorhandensein infektiöser Prionen nachzuweisen.

Bei der Entnahme des Gewebestücks wird insbesondere darauf geachtet, dass das entnomene Gewebestück frei von Blutgerinseln ist.

### Referenzliste

http://niah.naro.affrc.go.jp/disease/bse/retest_prionics.html

## Patentansprüche

1. Vorrichtung zum Abtrennen eines Gewebeteils aus Hirnmaterial umfassend eine kreisrunde Klinge, die sich als Wand eines zylindrischen oder im Wesentlichen zylindrischen Hohlkörpers fortsetzt, an dessen Ende ein senkrecht zur Zylinderachse angeordnetes Gitter angebracht ist.

2. Vorrichtung nach Anspruch 1, **dadurch gekennzeichnet, dass** die Vorrichtung zusätzlich einen Griff umfasst, der jenseits des Gitters am Zylinder der Klinge ansetzt und diesen in Richtung der Längsachse verlängert.

3. Vorrichtung nach Anspruch 2, **dadurch gekennzeichnet, dass** der Griff ein offener Hohlkörper ist.

4. Vorrichtung nach Anspruch 3, **dadurch gekennzeichnet, dass** der Griff eine Verbreiterung mit der Oberfläche eines Kegelstumpfes umfasst, dessen Enden kreisförmig sind, wobei das Ende mit dem geringeren Kreisdurchmesser jenseits des Gitters bündig am Zylinder der Klinge ansetzt und das Ende mit dem größeren Durchmesser bündig an einem zylindrischen oder im Wesentlichen zylindrischen Hohlkörper ansetzt.

5. Vorrichtung nach Anspruch 3, **dadurch gekennzeichnet, dass** der Griff folgendes umfasst: (a) einen ersten zylindrischen Hohlkörper, der jenseits des Gitters bündig am Zylinder der Klinge ansetzt und diesen in Richtung der Längsachse verlängert, (b) eine Verbreiterung mit der Oberfläche eines Kegelstumpfes, dessen Enden kreisförmig sind, wobei das Ende mit dem geringeren Kreisdurchmesser jenseits des Gitters am Zylinder von (a) ansetzt und das Ende mit dem größeren Durchmesser bündig an (c) einem zylindrischen oder im Wesentlichen zylindrischen Hohlkörper ansetzt.

6. Vorrichtung gemäß einem der Ansprüche 1 bis 5, **dadurch gekennzeichnet, dass** der Abstand zwischen Klingenkante und Gitter zwischen 5 mm und 20 mm beträgt.

7. Verwendung einer Vorrichtung gemäß einem der Ansprüche 1 bis 6 zum Abtrennen eines Gewebeteils aus Hirnmaterial.

8. Methode zum Abtrennen eines Gewebeteils aus Hirnmaterial, umfassend die Schritte:
(a) Bereitstellen eines Stückes Stammhirn enthaltend die Obex-Region, wobei das Stück Stammhirn mit der ventralen Seite auf eine glatte, feste Unterlage gelegt wird; gefolgt von
(b) senkrechtes Aufsetzen der Klinge einer Vorrichtung gemäß einem der Ansprüche 1 bis 6 auf einen vorbestimmten Ort der Obex-Region; gefolgt von
(c) Drücken der Klinge in senkrechter Richtung in das Hirnmaterial unter Durchführung von Drehbewegungen, bis die Klinge komplett Kontakt mit der Unterlage hat; gefolgt von
(d) Herausziehen der Vorrichtung aus dem Stück Stammhirn und Entnehmen des darin enthaltenen Gewebeteils.

9. Methode gemäß Anspruch 8, umfassend folgenden zusätzlichen Schritt nach Schritt (c):
(c') Drücken der Vorrichtung gegen die Unterlage und Anheben des Stückes Stammhirn, wobei zähe oder faserige Gewebebestandteile von dem von der Vorrichtung umschlossenen Gewebekern abgetrennt werden.

10. Ein Kit enthaltend eine oder mehrere Vorrichtungen gemäß einem der Ansprüche 1 bis 6, eine Gebrauchsvorschrift und Verpackungsmaterial enthaltend die vorgenannten Gegenstände.
